(19) Europäisches Patentamt
European Patent Office
Office européen des brevets

(11) Veröffentlichungsnummer: **0 199 331**
**B1**

(12) **EUROPÄISCHE PATENTSCHRIFT**

(45) Veröffentlichungstag der Patentschrift: **06.09.89**

(51) Int. Cl.⁴: **C 07 K 7/06**, C 07 K 5/10, A 61 K 37/02, G 01 N 33/53

(21) Anmeldenummer: **86105507.7**

(22) Anmeldetag: **21.04.86**

(54) **Pharmakologisch aktive Peptide.**

(30) Priorität: **23.04.85 DE 3514587**

(43) Veröffentlichungstag der Anmeldung: **29.10.86 Patentblatt 86/44**

(45) Bekanntmachung des Hinweises auf die Patenterteilung: **06.09.89 Patentblatt 89/36**

(84) Benannte Vertragsstaaten: **AT BE CH DE FR GB IT LI LU NL SE**

(56) Entgegenhaltungen:
**EP-A- 0 053 029**
**EP-A- 0 137 339**
**EP-A- 0 176 070**

**CHEMICAL ABSTRACTS, Band 103, 1985, Seite 85, Nr. 82014q, Columbus, Ohio, US; BRANTL, VICTOR et al.: "Novel opioid peptides derived from mitochondrial cytochrome b: cytochrophins" & EUR. J. PHARMACOL. 1985, 111(2), 293-4**
**UNLISTED DRUGS, Band 32, Nr. 10, Oktober 1980, Seite 149; beta-CasM-4**

(73) Patentinhaber: **Brantl, Victor, Dr. med., Dipl.-Chem., Hermann-Burte-Strasse 14, D-7846 Schliengen (DE)**

(72) Erfinder: **Brantl, Victor, Dr. med., Dipl.-Chem., Hermann-Burte-Strasse 14, D-7846 Schliengen (DE)**

(74) Vertreter: **Schacht, Wilhelm, Dr. jur., Am Eselsweg 47, D-6500 Mainz 1 (DE)**

**EP 0 199 331 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft pharmakologisch aktive Peptide, insbesondere opiatartig wirkend.

Aufgabe der Erfindung ist es, neue, bisher noch nicht beschriebene Peptide zu schaffen, die insbesondere opiatartig wirken.

Die Aufgabe ist gemäß der Erfindung dadurch gelöst, daß die Peptide folgende Struktur aufweisen:

Tyr-Pro-Phe-Thr-T
Tyr-Pro-Phe-Thr-A-T
Tyr-Pro-Phe-Thr-A-B-T
Tyr-Pro-Phe-Thr-A-B-C-T
Tyr-Pro-Phe-Thr-A-B-C-D-T
Tyr-Pro-Phe-Thr-A-B-C-D-E-T

wobei Tyr gleich der Aminosäure Tyrosin ist und das N-terminale L-Tyrosin der allgemeinen Formel

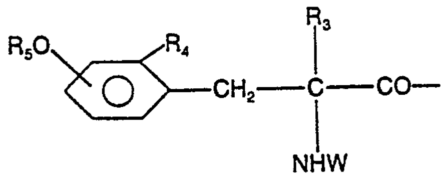

vorliegt, wobei im einzelnen steht

$R_3$ für Wasserstoff oder eine Alkylgruppe mit 1 bis 4 C-Atomen,

$R_4$ für Wasserstoff oder zusammen mit $R_3$ für eine Äthylenbrücke,

$R_5$ für Wasserstoff, eine Alkylgruppe mit 1 bis 4 C-Atomen oder eine $R_6CO$-Gruppe,

$R_6$ für einen gesättigten oder ungesättigten, geradkettigen oder verzweigten Alkylrest mit 1 bis 17 C-Atomen, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die $R_5O$-Gruppe sich in meta- oder para-Stellung zum

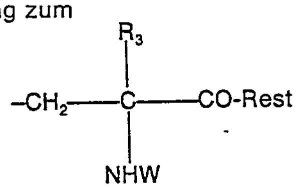

Rest befindet,

W für Wasserstoff, Alkyl mit 1 bis 5 C-Atomen Alkenyl mit 3 bis 5 C-Atomen, Cyclopropylmethyl, Cyclobutylmethyl, $R_6CO$-, H-Val, H-Ser, H-Arg, H-Lys, H-Ile, H-Tyr oder H-Phe;

Phe gleich der Aminosäure Phenylalanin ist, das auch in der allgemeinen Formel vorliegt

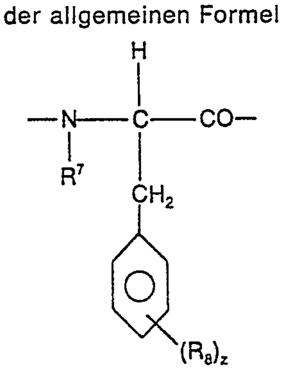

wobei im einzelnen bedeuten:

$R_7$ für Wasserstoff oder Alkyl mit 1 bis 4 C-Atomen,

$R_8$ für Wasserstoff, Fluor, Chlor, Brom, Nitro, Alkyl mit 1 bis 4 C-Atomen, z für 1 oder 2 steht.

Pro gleich der Aminosäure Prolin ist, das auch in der allgemeinen Formel vorliegt

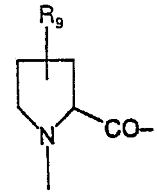

wobei im einzelnen bedeutet:

$R_9$ für Wasserstoff, eine Hydroxygruppe, eine Alkyl- oder Alkoxygruppe mit 1 oder 4 C-Atomen

– am Stickstoff eine Alkyl- oder Alkoxygruppe mit 1 bis 4 C-Atomen gebunden ist

– eine oder mehrere Ketogruppen in den Ring eingeführt sind;

und Thr gleich der Aminosäure Threonin ist. A, B, C, D, und E können jede beliebige Aminosäure der D- oder L-Form sein. T steht für OH, OR, $NH_2$, NHR, $NR_2$ oder NHNHR′, wobei R gegebenenfalls die folgende Bedeutung hat: Substit. lineares oder verzweigtes $C_{1-10}$-Alkyl, Adamantyl, $C_{1-10}$-Cycloalkyl oder $C_{6-8}$-Aralkyl, zweckmäßigerweise Phenyl, Benzyl oder Phenyläthyl bedeutet und R′ Wasserstoff, lineares oder verzweigtes $C_{1-10}$-Alkyl, Cycloalkyl oder $C_{6-8}$-Aralkyl, lineares, verzweigtes oder cyclisches aliphat. $C_{1-16}$-Acyl, gegebenenfalls durch OH, $NH_2$, $C_{1-14}$-Alkoxy oder Halogen substituiert, aromatisches Acyl, gegebenenfalls durch OH, $NH_2$, Halogen oder $C_{1-4}$-Alkoxy substituiert; lineares verzweigtes oder cyclisches $C_{3-11}$-aliphatisches Urethan darstellt und deren pharmazeutische annehmbare Salze.

Nach einer weiteren Ausbildung sind die Peptide dadurch gekennzeichnet, daß A = Isoleucin, Leucin, Serin oder Threonin, B = Isoleucin, Glycin, Serin oder Valin, C = Glycin, Leucin, Threonin, Asparagin oder Arginin, D = Glutamin, Lysin oder Arginin und E = Valin, Asparaginsäure, Leucin oder Arginin ist.

Nach einer anderen Weiterbildung der Erfindung liegen die Aminosäuren Prolin, Phenylalanin als Dehydroaminosäuren vor.

Nach einer besonders vorteilhaften Weiterbildung der Erfindung liegt das Phenylalanin in der 3. Aminosäureposition des Peptids (vom linken, N-terminalen Ende des Peptids her gerechnet) in der D-Form vor.

Dies hat insbesondere den Vorteil, daß die opiatartige Wirkung erhalten bleibt (zum Teil gesteigert wird) und eine besonders hohe Stabilität gegenüber peptidspaltenden Enzymen resultiert.

Den gleichen, o.g. stabilisierenden Effekt weisen die Peptide auf, wenn das Threonin der 4. Aminosäureposition in der D-Form vorliegt.

Die Aminosäure L-Threonin in der 4. Aminosäureposition kann nach einer Weiterbildung der Erfindung durch Glutamin, Glycin oder Asparaginsäure ersetzt sein.

Besonders vorteilhaft sind insbesondere Peptide folgender Struktur:

Tyr-Pro-Phe-Thr-OH
Tyr-Pro-Phe-Thr-$NH_2$
Tyr-Pro-Phe-Thr-Ile-OH
Tyr-Pro-Phe-Thr-Ile-Ile-OH
Tyr-Pro-Phe-Thr-Ile-Ile-Gly-OH
Tyr-Pro-Phe-Thr-Ile-Ile-Gly-Gln-OH
Tyr-Pro-Phe-Thr-Ile-Ile-Gly-Gln-Val-OH
Tyr-Pro-D-Phe-Thr-OH
Tyr-Pro-D-Phe-Thr-$NH_2$
Tyr-Pro-D-Phe-Thr-Ile-OH
Tyr-Pro-D-Phe-Thr-Thr-OH
Tyr-Pro-Phe-D-Thr-Thr-$NH_2$
Tyr-Pro-Phe-Gln-OH
Tyr-Pro-Phe-Gln-$NH_2$
Tyr-Pro-Phe-Gly-$NH_2$
Tyr-Pro-Phe-Asp-$NH_2$
Tyr-Pro-Phe-Thr-Thr-OH
Tyr-Pro-Phe-Thr-Ser-OH
Tyr-Pro-Phe-Thr-Leu-OH

Die erfindungsgemäßen Peptide können Wirkungen auf das zentrale Nervensystem ausüben. Das kann zum Beispiel eine schmerzstillende (analgetische) Wirkung sein; die analgetische Wirkung kann ganz oder teilweise über opioide Rezeptoren vermittelt werden.

Weiterhin können die erfindungsgemäßen Peptide endokrine Wirkungen auslösen, z.B. eine Erhöhung des Wachstumhormonspiegels.

Die erfindungsgemäßen Peptide können als Immunomodulatoren Anwendung finden. Die Verabreichung solcher Peptide führt z.B. bei einem geschwächten Organismus zu einer Stimulation des körpereigenen Immunsystems.

Die Substanzen können auch zur Aktivierung des Zellstoffwechselsystems dem Säugetierorganismus zugeführt werden; hierbei wird die Sauerstoffaufnahme der Gewebszellen erhöht, dies kann insbesondere bei altersbedingten Krankheiten von Bedeutung sein.

Die erfindungsgemäßen Peptide können nach Kopplung an Thyreoglobulin oder andere makromolekulare Eiweißkörper als Antigene zur Erzeugung von Antikörpern im Säugetierorganismus verwendet werden. Hierbei wird das Peptid in üblicher Weise mit Carbodiimid an das Makromolekül gekoppelt und intracutan in die Rücken- und Bauchhaut von Kaninchen injiziert.

Die Antikörper gegen die erfindungsgemäßen Peptide können zur Bestimmung derselben in Körpergeweben und -Flüssigkeiten verwendet werden. Dies kann sowohl zur Bestimmung exogen zugeführter, wie auch endogen entstandener Peptide verwendet werden (Diagnostik).

Die erfindungsgemäßen Peptide und/oder deren Derivate und/oder deren Salze können in Human- und Tierarzneimitteln enthalten sein. Diese können insbesondere als Antitussiva, Antidiarrhoika, Modulatoren des Immunsystems, Analgetika, Antipsychotika, Tranquilizer, Durchblutungsförderer und zur Förderung der Wundheilung Verwendung finden.

Beispiel:

1) Synthese zweier erfindungsgemäßer Peptide folgender Sequenz:

Tyr-Pro-Phe-Thr-OMet/-OH
Tyr-Pro-Phe-Thr-Ile-OMet/-OH

Die verwendete Synthesemethode benützt Benzyloxycarbonyl-geschützte Aminosäuren als gemischte Anhydride (Lottspeich et al., Hoppe-Seyler's Z. Physiol. Chem., 1835–1839, 1980).

Hierbei wird die Aminokomponente bei −15 °C oder niedriger in Dimethylformamid (DMF) mit einem 0,5 molaren Überschuß an gemischtem Anhydrid einer Z-Aminosäureisobutylcarbonsäure (wobei Z– als Schutzgruppe dient und einen N-Benzyloxycarbonyl-Rest darstellt) 2–4 Stunden umgesetzt. Nach der 2–4 stündigen Kupplung wird der Überschuß an Anhydrid zerstört. Bei 0 °C wird dann der pH-Wert des Reaktionsproduktes mit wässriger, gesättigter $KHCO_3$-Lösung auf 8 eingestellt und 30 Minuten bei 0 °C gerührt. Die Peptide werden mit Äthylacetat extrahiert. Das Äthylacetat/Peptidgemisch wird, um das Z-Aminosäure-Kaliumsalz zu entfernen, 3 mal mit NaCL/Wasser, 3 mal mit Wasser gewaschen und abgedampft. Das so erhaltene Peptid, welches noch die Schutzgruppe Z trägt, wird dann in Methanol hydriert. Hierbei werden 100–500 mg Pd/Aktivkohle als Katalysator pro mmol Peptid zugesetzt. Die $CO_2$-Abspaltung wird mit $Ba(OH)_2$-Lösung kontrolliert. Der Katalysator wird dann abfiltriert (Papierfilter), mit Wasser ausgiebig gewaschen und das Filtrat auf einem Rotationsverdampfer eingedampft. Das gewünschte deblockierte Peptid ist im Rückstand enthalten.

I. Herstellung des Zwischenproduktes L-Pro-L-Phe-L-Thr-OMet (I)

I/Stufe 1a) Herstellung des gemischten Anhydrids: Z-Pro-Phe-gem. Anhydrid

592,5 mg (1,5 mmol, = 40%iger Überschuß) des Dipeptids Z-Pro-Phe (Bachem, Schweiz) werden in 20 ml DMF mit 200 µl (1,5 mmol) Chlorameisensäureisobutylester bei −15 °C nach Zusatz von 170 µl (1,5 mmol) N-Methylmorpholin 15 Minuten umgesetzt.

I/Stufe 1b) Vorbereitung der Aminokomponente.

170 mg (1,0 mmol) Threoninmethylesterhydrochlorid (Novabiochem, Schweiz) werden in 20 ml DMF unter Zusatz von 110 µl (1,0 mmol) N-Methylmorphin bei −15 °C aufgelöst.

I/Stufe 2) Umsetzung des gemischten Anhydrids der Stufe I/1a mit der Aminokomponente der Stufe I/1b.

Das Z-Pro-Phe-gem. Anhydrid wird mit dem Thr-OMet in insgesamt 40 ml DMF bei −15 °C 4 Stunden zum Z-Pro-Phe-Thr-OMet umgesetzt.

Vor der Aufarbeitung wird der 50%ige Überschuß an gemischtem Anhydrid zerstört. Bei 0 °C wird der pH-Wert des Reaktionsproduktes mit wässriger, gesättigter $KHCO_3$-Lösung auf pH 8 eingestellt und 30 Minuten bei 0 °C gerührt. Danach wird das Peptid mit 50–100 ml Äthylacetat (EtAc) extrahiert; das EtAc/Peptidgemisch wird mit ge-

sättigter, wässriger NaCl-Lösung gewaschen. Nach einem weiteren abschließenden Waschen mit Wasser wird die EtAc-Phase eingedampft.

I/Stufe 3) Abspalten der Schutzgruppe durch Hydrierung.

Das Peptid wird in 30 ml Methanol gelöst und 100 mg Palladium auf Aktivkohle (Merck) zugegeben. Nach dem Verdrängen der Luft durch Stickstoff wird in das Reaktionsgefäß Wasserstoff eingeleitet. Die Hydrierung wird bei 25°–30 °C durchgeführt. Die Hydrierung ist beendet, wenn kein $CO_2$ mehr freigesetzt wird, d.h. wenn nach Kontrolle in wässriger $Ba(OH)_2$-Lösung kein Niederschlag mehr gebildet wird. Die Lösung wird filtriert, mit Wasser gewaschen und am Rotationsverdampfer einrotiert. Das bleibende Produkt Pro-Phe-Thr-OMet (I) wird dann später als Aminokomponente eingesetzt.

II. Herstellung des Zwischenproduktes L-Pro-L-Phe-L-Thr-L-Ile-OMet (II)

II/Stufe 1a) Herstellung des gemisches Anhydrids: Z-Thr-gem. Anhydrid.

379,5 mg (1,5 mmol) Z-L-Thr (Novabiochem, Schweiz) werden in 20 ml DMF unter Zusatz von 170 µl (1,5 mmol) N-Methylmorpholin gelöst und mit 180 µl Chlorameisensäureisobutylester bei −15 °C 15 Minuten umgesetzt.

II/Stufe 1b) Vorbereitung der Aminokomponente.

185 mg (1,0 mmol) L-Isoleucin-Methylesterhydrochlorid (Bachem, Schweiz) werden in 20 ml DMF unter Zusatz von 170 µl (1,5 mmol) N-Methylmorpholin gelöst.

II/Stufe 2) Umsetzung des gem. Anhydrids der Stufe II/1a mit der Aminokomponente der Stufe II/1b.

Das Z-Thr-gem. Anhydrid wird mit dem Ile-OMet in insgesamt 40 ml DMF bei −15 °C 4 Stunden zum Z-Thr-Ile-OMet umgesetzt.

Die weitere Aufarbeitung erfolgt analog wie bereits unter I/2 und I/3 beschrieben. Schließlich erhält man den Dipeptidmethylester Thr-Ile-OMet, der nachfolgend als Aminokomponente weiter umgesetzt wird.

II/Stufe 3a) Herstellung des gemischten Anhydrids: Z-Pro-Phe-gem. Anhydrid

Der gleiche Ansatz wird wie unter Stufe I/1a) beschrieben hergestellt.

II/Stufe 3b) Vorbereitung der Aminokomponente.

Das in Stufe II/2 erhaltene Dipeptid wird in 20 ml DMF unter Zusatz von 170 µl (1,5 mmol) N-Methylmorpholin gelöst.

II/Stufe 4) Umsetzung des gemischten Anhydrids der Stufe II/3a) mit der Aminokomponente der Stufe II/3b

Das Z-Pro-Phe-gem. Anhydrid wird mit dem Thr-Ile-OMet in 40 ml DMF bei −15 °C 4 Stunden zum Z-Pro-Phe-Thr-Ile-OMet umgesetzt.

Die weitere Aufarbeitung erfolgt analog wie bereits unter I/2 und I/3 beschrieben. Das erhaltene Tetrapeptid Pro-Phe-Thr-Ile-OMet (II) wird dann später als Aminokomponente eingesetzt.

III. Weiterverarbeitung der Zwischenprodukte
Pro-Phe-Thr-OMet (I)
Pro-Phe-Thr-Ile-OMet (II)

III/Stufe 1a) Herstellung des gem. Anhydrids: Z-Tyr-gem. Anh.

674 mg (1,5 mmol) N,O-di-Z-Tyrosin (Novabiochem, Schweiz) werden in 20 ml DMF unter Zusatz von 170 µl (1,5 mmol) N-Methylmorpholin gelöst und mit 200 µl (1,5 mmol) Chlorameisensäureisobutylester bei −15 °C 15 Minuten umgesetzt.

III/Stufe 1b)

Die Zwischenprodukte I und II werden in 20 ml DMF gelöst und mit je einem Ansatz der Stufe III/1a bei −15 °C 4 Stunden umgesetzt.

III/Stufe 2)

Die Aufarbeitung der jeweiligen Reaktionsprodukte der Stufe III/1b erfolgt wie unter I/2 und I/3 beschrieben. Die Syntheseendprodukte

Tyr-Pro-Phe-Thr-OMet
Tyr-Pro-Phe-Thr-Ile-OMet

werden dann entweder direkt über Säulenchromatographie (Biogel P 2) gereinigt (bzw. nach einer in üblicher Weise durchgeführten sauren Esterhydrolyse) und nachfolgend analysiert, so wie von Lottspeich et al. (Hoppe-Seyler's Z. Physiol. Chem., 361, 1835–1839, 1980) beschrieben. Die gereinigten und mittels Aminosäureanalyse analysierten Peptide

Tyr-Pro-Phe-Thr-OH
Tyr-Pro-Phe-Thr-Ile-OH

wurden dann auf pharmakologische Wirkungen hin untersucht. (Vgl. Beispiel, 2)

Die Herstellung der anderen erfindungsgemäßen Peptide erfolgte auf die gleiche Weise, wie dies oben bereits beschrieben wurde, wobei entsprechend der gewünschten Sequenz andere Z-Aminosäurederivate eingesetzt werden, so z.B. statt Z-L-Threonin, Z-D-Threonin; statt Z-Pro-Phe, Z-Pro-D-Phe; bzw. andere Aminosäuremethylester z.B. Threoninmethylester oder Serinmethylester verwendet werden. Die Synthese der anderen erfindungsgemässen Peptide erfolgt ebenfalls analog den Syntheseverfahren wie Lottspeich et al. (1980) beschrieben (siehe oben).

Die Peptidester wurden zum Zwecke der C-terminalen Amidierung in üblicher Weise einer Ammonolyse unterworfen.

2) Pharmakologische Wirkungen zweier erfindungsgemäßer Peptide (Tyr-Pro-Phe-Thr-OH und Tyr-Pro-Phe-Thr-Ile-OH)

Die beiden Substanzen zeigen spezifische opiatartige Wirkungen am elektrisch stimulierten plexus myentericus/Längsmuskel-Präparat des Meerschweinchenileums (GPI), Methode nach

Schulz und Goldstein, J. Pharmacol. Exptl. Ther. 183, 400, 1972.

Die Ergebnisse sind in Tabelle 1 aufgeführt.

Tabelle 1
Opiatartige Wirkungen zweier erfindungsgemässer Peptide; die Zahlen geben diejenige Konzentration (µM) an, die notwendig ist, die elektrisch induzierte Kontraktion des Meerschweinchen-Darm-Präparates (GPI) um 50% zu hemmen ($IC_{50}$-Wert). Die Zahlen sind Mittelwerte aus 4–5 Bestimmungen; die Standardabweichung von den Mittelwerten ist kleiner als 13%. Die Hemmungen des GPI sind mit dem spezifischen Opioid-Antagonisten Naloxon aufhebbar bzw. bei Vorbehandlung blockierbar.

| Substanz | GPI |
|---|---|
| Tyr-Pro-Phe-Thr | 120,1 |
| Tyr-Pro-Phe-Thr-Ile | 290,1 |
| Normorphin | 0,1 |

Die entsprechenden D-$Ala^2$-Verbindungen zeigen noch stärkere opiatartige Wirkungen am GPI-Präparat.

Opiatartige Wirkungen können auch nach intracerebroventrikulärer Injektion bei Ratten beobachtet werden. Hierbei wird so vorgegangen, wie von Brantl et al., Life Sciences, 28, 1903–1909, 1981 beschrieben wurde. Als wirksame analgetische Dosis erwiesen sich 400 bis 800 µg der o.g. Peptide; die analgetische Wirkung ist mit Naloxon 10 mg pro kg Körpergewicht (intraperitoneal) aufhebbar bzw. bei Vorbehandlung mit Naloxon blockierbar.

**Patentansprüche**

1. Pharmakologisch aktive Peptide der Formel:
Tyr-Pro-Phe-Thr-T
Tyr-Pro-Phe-Thr-A-T
Tyr-Pro-Phe-Thr-A-B-T
Tyr-Pro-Phe-Thr-A-B-C-T
Tyr-Pro-Phe-Thr-A-B-C-D-T
Tyr-Pro-Phe-Thr-A-B-C-D-E-T
wobei Tyr gleich der Aminosäure Tyrosin ist und das N-terminale L-Tyrosin der allgemeinen Formel:

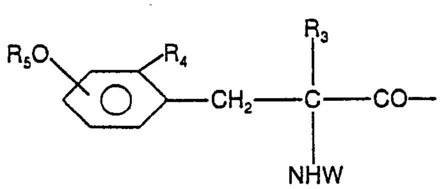

vorliegt, wobei im einzelnen steht

$R_3$ für Wasserstoff oder eine Alkylgruppe mit 1 bis 4 C-Atomen,

$R_4$ für Wasserstoff oder zusammen mit $R_3$ für eine Äthylenbrücke,

$R_5$ für Wasserstoff, eine Alkylgruppe mit 1 bis 4 C-Atomen oder eine $R_6CO$-Gruppe,

$R_6$ für einen gesättigten oder ungesättigten, geradkettigen oder verzweigten Alkylrest mit 1 bis 17 C-Atome, einen Phenylrest oder einen Phenylalkylrest mit 7 bis 12 C-Atomen, wobei die Phenylreste durch 1 oder 2 Substituenten aus der Reihe Halogen, Alkyl mit 1 bis 4 C-Atomen oder Alkoxy mit 1 bis 4 C-Atomen substituiert sein können, wobei die $R_5O$-Gruppe sich in meta- oder para-Stellung zum

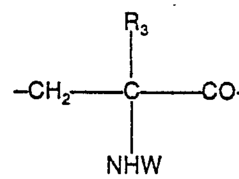
Rest befindet,

W für Wasserstoff, Alkyl mit 1 bis 5 C-Atomen Alkenyl mit 3 bis 5 C-Atomen, Cyclopropylmethyl, Cyclobutylmethyl, $R_6CO$–, H-Val, H-Ser, H-Arg, H-Lys, H-Ile, H-Tyr oder H-Phe;

Phe gleich der Aminosäure Phenylalanin ist, das auch in der allgemeinen Formel vorliegt

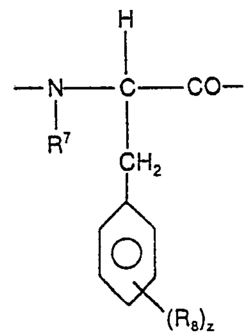

wobei im einzelnen bedeuten:

$R_7$ für Wasserstoff oder Alkyl mit 1 bis 4 C-Atomen,

$R_8$ für Wasserstoff, Fluor, Chlor, Brom, Nitro, Alkyl mit 1 bis 4 C-Atomen, z für 1 oder 2 steht;

Pro gleich der Aminosäure Prolin ist, das auch in der allgemeinen Formel vorliegt

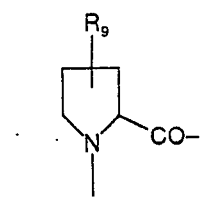

wobei im einzelnen bedeutet:

$R_9$ für Wasserstoff, eine Hydroxygruppe, eine Alkyl- oder Alkoxygruppe mit 1 oder 4 C-Atomen

– am Stickstoff eine Alkyl- oder Alkoxygruppe mit 1 bis 4 C-Atomen gebunden ist

– eine oder mehrere Ketogruppen in den Ring eingeführt sind;

und Thr gleich der Aminosäure Threonin ist. A, B, C, D, und E können jede beliebige Aminosäure der D- oder L-Form sein. T steht für OH, OR, $NH_2$, NHR, $NR_2$ oder NHNHR′, wobei R gegebenenfalls die folgende Bedeutung hat: Substit. lineares oder verzweigtes $C_{1-10}$-Alkyl, Adamantyl, $C_{1-10}$-Cycloalkyl oder $C_{6-8}$-Aralkyl, zweckmäßigerweise Phenyl,

Benzyl oder Phenyläthyl bedeutet und R′ Wasserstoff, lineares oder verzweigtes $C_{1-10}$-Alkyl, Cycloalkyl oder $C_{6-8}$-Aralkyl, lineares, verzweigtes oder cyclisches aliphat. $C_{1-16}$-Acyl, gegebenenfalls durch OH, $NH_2$, $C_{1-14}$-Alkoxy oder Halogen substituiert, aromatisches Acyl, gegebenenfalls durch OH, $NH_2$, Halogen oder $C_{1-4}$-Alkoxy substituiert; lineares verzweigtes oder cyclisches $C_{3-11}$ aliphatisches Urethan darstellt und deren pharmazeutische annehmbare Salze.

2. Pharmakologisch aktive Peptide nach Anspruch 1, dadurch gekennzeichnet, daß A = Isoleucin, Leucin, Serin oder Threonin, B = Isoleucin, Glycin, Serin oder Valin, C = Glycin, Leucin, Threonin, Asparagin oder Arginin, D = Glutamin, Lysin oder Arginin und E = Valin, Asparaginsäure, Leucin oder Arginin ist.

3. Pharmakologisch aktive Peptide nach Anspruch 1 und 2, dadurch gekennzeichnet, daß die Aminosäuren Prolin und Phenylalanin als Dehydroaminosäuren vorliegen.

4. Pharmakologisch aktive Peptide nach Anspruch 1–3, dadurch gekennzeichnet, daß das Phenylalanin in der 3. Aminosäureposition des Peptids (vom linken, N-terminalen Ende des Peptids her gerechnet) in der D-Form vorliegt.

5. Pharmakologisch aktive Peptide nach Anspruch 1–4, dadurch gekennzeichnet, daß das Threonin der 4. Aminosäureposition in der D-Form vorliegt.

6. Pharmakologisch aktive Peptide nach Anspruch 1–5, dadurch gekennzeichnet, daß die Aminosäure L-Threonin in der 4. Aminosäureposition durch Glutamin, Glycin oder Asparaginsäure ersetzt ist.

7. Pharmakologisch aktive Peptide nach Anspruch 1–6, dadurch gekennzeichnet, daß die Peptide folgende Struktur aufweisen:

Tyr-Pro-Phe-Thr-OH
Tyr-Pro-Phe-Thr-$NH_2$
Tyr-Pro-Phe-Thr-Ile-OH
Tyr-Pro-Phe-Thr-Ile-Ile-OH
Tyr-Pro-Phe-Thr-Ile-Ile-Gly-OH
Tyr-Pro-Phe-Thr-Ile-Ile-Gly-Gln-OH
Tyr-Pro-Phe-Thr-Ile-Ile-Gly-Gln-Val-OH
Tyr-Pro-D-Phe-Thr-OH
Tyr-Pro-D-Phe-Thr-$NH_2$
Tyr-Pro-D-Phe-Thr-Ile-OH
Tyr-Pro-D-Phe-Thr-Thr-OH
Tyr-Pro-Phe-D-Thr-Ile-OH
Tyr-Pro-Phe-D-Thr-Ile-$NH_2$
Tyr-Pro-Phe-D-Thr-Thr-OH
Tyr-Pro-Phe-D-Thr-Thr-$NH_2$
Tyr-Pro-Phe-Gln-OH
Tyr-Pro-Phe-Gln-$NH_2$
Tyr-Pro-Phe-Gly-$NH_2$
Tyr-Pro-Phe-Asp-OH
Tyr-Pro-Phe-Asp-$NH_2$
Tyr-Pro-Phe-Thr-Thr-OH
Tyr-Pro-Phe-Thr-Ser-OH
Tyr-Pro-Phe-Thr-Leu-OH

8. Pharmakologisch aktive Peptide nach Anspruch 1–7, dadurch gekennzeichnet, daß diese Wirkungen auf das zentrale Nervensystem ausüben.

9. Pharmakologisch aktive Peptide nach Anspruch 1–7, dadurch gekennzeichnet, daß diese endokrine Wirkungen auslösen.

10. Pharmakologisch aktive Peptide nach Anspruch 1–7, dadurch gekennzeichnet, daß die Peptide immunmodulierende Wirkungen vermitteln.

11. Pharmakologisch aktive Peptide nach Anspruch 1–7, dadurch gekennzeichnet, daß die Peptide eine Aktivierung des Zellstoffwechsels bewirken.

12. Pharmakologisch aktive Peptide nach Anspruch 1–7, dadurch gekennzeichnet, daß diese nach Kopplung an Thyreoglobulin oder andere makromolekulare Eiweißkörper als Antigene zur Erzeugung von Antikörpern im Säugetierorganismus verwendet werden.

13. Pharmakologisch aktive Peptide nach Anspruch 1–7, dadurch gekennzeichnet, daß die Antikörper gegen die erfindungsgemäßen Peptide zur Bestimmung derselben in Körpergeweben und -Flüssigkeiten verwendet werden.

14. Arzneimittel und Tierarzneimittel, dadurch gekennzeichnet, daß sie die in den Ansprüchen 1–11 charakterisierten pharmakologisch aktiven Peptide und/oder deren Derivate und/oder deren Säureadditionssalze und/oder deren Metallkomplexe enthalten.

**Claims**

1. Pharmacologically active peptides of the formula:

Tyr-Pro-Phe-Thr-T
Tyr-Pro-Phe-Thr-A-T
Tyr-Pro-Phe-Thr-A-B-T
Tyr-Pro-Phe-Thr-A-B-C-T
Tyr-Pro-Phe-Thr-A-B-C-D-T
Tyr-Pro-Phe-Thr-A-B-C-D-E-T

wherein Tyr is equal to the amino acid tyrosine and the N-terminal L-tyrosine of the general formula

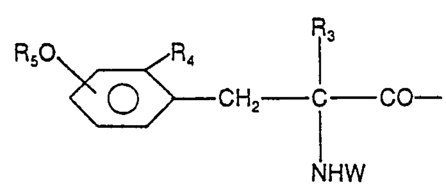

is present wherein the following individually signify:

$R_3$ represents hydrogen or an alkyl group with 1 to 4 C-atoms,

$R_4$ represents hydrogen or, together with $R_3$, an ethylene bond,

$R_5$ represents hydrogen, an alkyl group with 1 to 4 C-atoms or a $R_6CO$-group,

$R_6$ represents a saturated or unsaturated, straight chain or branched chain alkyl residue with 1 to 17 C-atoms, a phenyl residue or a phenylalkyl residue with 7 to 12 C-atoms, whereby the phenyl residues can be substituted by 1 or 2 substituents from the halogen series, alkyl with 1 to 4 C-atoms or alkoxy with 1 to 4 C-atoms, whereby the $R_5O$-group is in the meta- or para position to

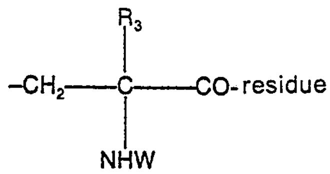

in which W represents hydrogen, alkyl with 1 to 5 C-atoms, alkenyl with 3 to 5 C-atoms, cyclopropylmethyl, cyclobutylmethyl, $R_6CO-$, H-Val, H-Ser, H-Arg, H-Lys, H-Ile, H-Tyr or H-Phe;

Phe is equal to the amino acid phenylalanine which is present also in the general formula:

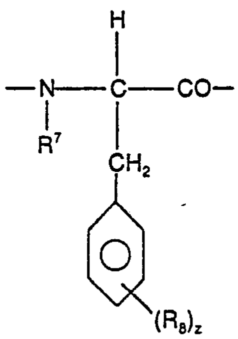

in which

$R_7$ represents hydrogen or alkyl with 1 to 4 C-atoms,

$R_8$ represents hydrogen, fluorine, chlorine, bromine, nitro, alkyl with 1 to 4 C-atoms, z stands for 1 or 2;

Pro is equal to the amino acid proline which is present also in the general formula:

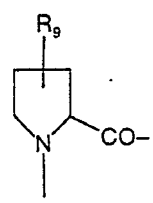

wherein:

$R_9$ represents hydrogen, a hydroxy group, an alkyl- or alkoxy group with 1 or 4 C-atoms

– at the nitrogen, an alkyl or alkoxy group with 1 to 4 C-atoms is combined

– one or several keto groups are introduced into the ring,

and Thr is equal to the amino acid threonine;

A, B, C, D, and E can be any amino acid of the D- or L-form. T represents OH, OR, $NH_2$, NHR, $NR_2$, or NHNHR′, wherein R, if necessary, has the following significance: substituted linear or branched $C_{1-10}$-alkyl, adamantyl, $C_{1-10}$-cycloalkyl or $C_{6-8}$-aralkyl advantageously phenyl, benzyl or phenylethyl and R′ signifies hydrogen, linear or branched $C_{1-10}$-alkyl, cycloalkyl or $C_{6-8}$-aralkyl, linear, branched or cyclic aliphatic $C_{1-16}$-acyl, substituted, if necessary, by OH, $NH_2$, $C_{1-14}$-alkoxy or halogen; aromatic acyl, substituted, if necessary, by OH, $NH_2$, halogen or $C_{1-4}$-alkoxy; linear, branched or cyclic $C_{3-11}$-aliphatic urethane and their pharmaceutically acceptable salts.

2. Pharmacologically active peptides according to claim 1, characterized in that the following signify: A = isoleucine, leucine, serine or threonine, B = isoleucine, glycine, serine or valine, C = glycine, leucine, threonine, asparagine or arginine, D = glutamine, lysine or arginine and E = valine, asparaginic acid, leucine or arginine.

3. Pharmacologically active peptides according to claims 1 and 2, characterized in that the amino acids proline and phenylalanine are present as de-hydroamino acids.

4. Pharmacologically active peptides according to claims 1 to 3, characterized in that the phenylalanine is present in the third amino acid position of the peptide (calculated from the left N-terminal end of the peptide) in the D-form.

5. Pharmacologically active peptides according to claims 1 to 4, characterized in that the threonine of the fourth amino acid position is present in the D-form.

6. Pharmacologically active peptides according to claims 1 to 5, characterized in that the amino acid L-threonine in the fourth amino acid position is replaced by glutamine, glycine or asparaginic acid.

7. Pharmacologically active peptides according to claims 1 to 6, characterized in that the peptides have the following structure:

Tyr-Pro-Phe-Thr-OH
Tyr-Pro-Phe-Thr-$NH_2$
Tyr-Pro-Phe-Thr-Ile-OH
Tyr-Pro-Phe-Thr-Ile-Ile-OH
Tyr-Pro-Phe-Thr-Ile-Ile-Gly-OH
Tyr-Pro-Phe-Thr-Ile-Ile-Gly-Gln-OH
Tyr-Pro-Phe-Thr-Ile-Ile-Gly-Gln-Val-OH
Tyr-Pro-D-Phe-Thr-OH
Tyr-Pro-D-Phe-Thr-$NH_2$
Tyr-Pro-D-Phe-Thr-Ile-OH
Tyr-Pro-D-Phe-Thr-Thr-OH
Tyr-Pro-Phe-D-Thr-Ile-OH
Tyr-Pro-Phe-D-Thr-Ile-$NH_2$
Tyr-Pro-Phe-D-Thr-Thr-OH
Tyr-Pro-Phe-D-Thr-Thr-$NH_2$
Tyr-Pro-Phe-Gln-OH
Tyr-Pro-Phe-Gln-$NH_2$
Tyr-Pro-Phe-Gly-$NH_2$
Tyr-Pro-Phe-Asp-OH
Tyr-Pro-Phe-Asp-$NH_2$
Tyr-Pro-Phe-Thr-Thr-OH
Tyr-Pro-Phe-Thr-Ser-OH
Tyr-Pro-Phe-Thr-Leu-OH

8. Pharmacologically active peptides according to claims 1 to 7, characterized in that they effect on the central nervous system.

9. Pharmacologically active peptides according to claims 1 to 7, characterized in that they trigger endocrine effects.

10. Pharmacologically active peptides according to claims 1 to 7, characterized in that the peptides transmit immune modulating effects.

11. Pharmacologically active peptides according to claims 1 to 7, characterized in that the peptides effect an activation of the cell metablism.

12. Pharmacologically active peptides according to claims 1 to 7, characterized in that they, after

coupling to the thyreoblobulin or other macromolecular albumins, are used as antigens for the production of antibodies in the mammalian organism.

13. Pharmacologically active peptides according to claims 1 to 7, characterized in that the antibodies are used against the peptides according to this invention for the analysis of the same in body tissues and body fluids.

14. Human and animal medication, characterized in that they contain pharmacologically active peptides, and/or their derivatives, and/or their acid addition salts, and/or their metal complexes, which were characterized in the claims 1 to 11.

**Revendications**

1. Peptides à activité pharmacologique des formules suivantes:

Tyr-Pro-Phe-Thr-T
Tyr-Pro-Phe-Thr-A-T
Tyr-Pro-Phe-Thr-A-B-T
Tyr-Pro-Phe-Thr-A-B-C-T
Tyr-Pro-Phe-Thr-A-B-C-D-T
Tyr-Pro-Phe-Thr-A-B-C-D-E-T

dans lesquelles

Tyr représente le résidu d'amino-acide tyrosine et la L-tyrosine N-terminale de formule générale est présent

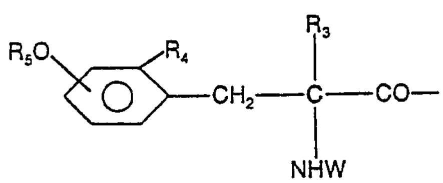

En ce formule générale représente

$R_3$ un atome d'hydrogène ou un groupe alkyle en $C_1$ à $C_4$,

$R_4$ un atome d'hydrogène ou forme avec $R_3$ un pont éthylène,

$R_5$ un atome d'hydrogène, un groupe alkyle en $C_1$ à $C_4$ ou un groupe $R_6CO$–,

$R_6$ un résidu d'alkyle saturé ou non saturé, linéaire ou ramifié comportant 1 à 17 atomes de carbone, un reste phényle ou phénylalkyle comportant 7 à 12 atomes de carbone, les restes phényles pouvant être substitués par 1 ou 2 substituants de la série des halogènes, des restes alkyles en $C_1$ à $C_4$ ou d'alcoxy en $C_1$ à $C_4$, le groupe $R_5O$ étant en position méta- ou para- par rapport au reste

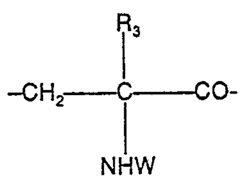

et

W étant un atome d'hydrogène, un alkyle en $C_1$ à $C_5$, un alkényle en $C_3$ à $C_5$, un cyclopropylméthyle, un cyclobutylméthyle, un groupe $R_6CO$–, les résidus d'aminoacides H-Val, H-Ser, H-Arg, H-Lys, H-Ile, H-Tyr ou H-Phe;

Phe représente le résidu d'amino acide phénylalanine étant aussi de formule générale

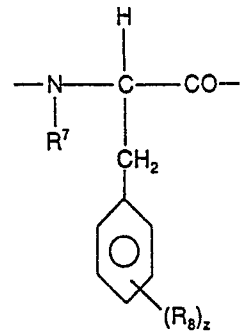

dans laquelle

$R_7$ représente un atome d'hydrogène ou un groupe alkyle en $C_1$ à $C_4$,

$R_8$ représente un atome d'hydrogène, -de fluore, -de chlore, -de brome, un groupe nitro, un groupe alkyle en $C_1$ à $C_4$ et,

Z représentant 1 ou 2;

Pro représente le résidu d'amino acide proline étant aussi de formule générale

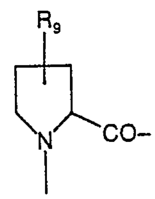

En ce formule générale représente

$R_9$ un atome d'hydrogène, un groupement hydroxy, un groupe alkyle ou un groupe alcoxy en $C_{1-4}$,

– l'atome d'azote est lié à un groupe alkyle ou un groupe alcoxy comportant 1 à 4 atomes de carbone,

– un ou plusieurs groupes céto sont introduits dans le cycle;

et

Thr représente le résidu d'amino acide threonine;

A, B, C, D et E peuvent être chaques quelconques résidus d'amino acides de la configuration stéréochimique D ou L;

T représente OH, OR, $NH_2$, NHR, $NR_2$ ou NHNHR', R éventuellement représentant

– un groupe alkyle en $C_{1-10}$ linéaire ou ramifié et substitué, un groupe adamantyle, un groupe cycloalkyle en $C_{1-10}$ ou un groupe aralkyle en $C_{6-8}$, avantageusement un groupe phényle, un groupe benzyle ou un groupe phényléthyle;

et R' représente

– un atome d'hydrogène, un groupe alkyle en $C_{1-10}$ linéaire ou ramifié, un groupe cycloalkyle ou un groupe aralkyle en $C_{6-8}$, un groupement acyle en $C_{1-16}$ aliphatique, linéaire, ramifié ou cyclique, éventuellement substitué par OH, $NH_2$, un groupe alcoxy en $C_{1-14}$ ou halogène,

– un groupe acyle aromatique, éventuellement substitué par OH, $NH_2$, halogène ou un groupe alcoxy en $C_{1-4}$,

– un groupe de type uréthane aliphatique en $C_{3-11}$ linéaire, ramifié ou cyclique
et de même que
les sels pharmaceutiquement acceptables des substances que l'ont vient de mentionner.

2. Peptides à activité pharmacologique selon la revendication 1, caractérisés en ce que
A représente les résidus d'amino acides isoleucine, leucine, serine ou threonine,
B représente les résidus d'amino acides isoleucine, glycine, serine ou valine,
C représente les résidus d'amino acides glycine, leucine, threonine, asparagine ou arginine,
D représente les résidus d'amino acides glutamine, lysine ou arginine,
E représente les résidus d'amino acides valine, leucine, arginine ou acide de asparagine.

3. Peptides à activité pharmacologique suivant l'une des revendications 1 et 2, caractérisés en ce que les amino acides proline et phénylalanine se présentent sous forme de déhydroamino-acides.

4. Peptides à activité pharmacologique, suivant l'une des revendications 1–3, caractérisés en ce que la phénylalanine occupant la troisième position d'amino acide du peptide (calculé de bout N-terminal) se présente dans la configuration stéréochimique D.

5. Peptides à activité pharmacologique, suivant l'une des revendications 1–4, caractérisés en ce que la threonine occupant la quatrième position d'amino acide du peptide (calculé de bout N-terminal) se présente dans la configuration stéréochimique D.

6. Peptides à activité pharmacologique, suivant l'une des revendications 1–5, caractérisés en ce que l'amino acide L-threonine occupant la quatrième position d'amino acide du peptide (calculé de bout N-terminal) est substitué par glutamine, glycine ou acide de asparagine.

7. Peptides à activité pharmacologique, suivant l'une des revendications 1–6, caractérisés en ce qu'ils possèdent les structures suivantes:
Tyr-Pro-Phe-Thr-OH
Tyr-Pro-Phe-Thr-$NH_2$
Tyr-Pro-Phe-Thr-Ile-OH
Tyr-Pro-Phe-Thr-Ile-Ile-OH
Tyr-Pro-Phe-Thr-Ile-Ile-Gly-OH
Tyr-Pro-Phe-Thr-Ile-Ile-Gly-Gln-OH
Tyr-Pro-Phe-Thr-Ile-Ile-Gly-Gln-Val-OH
Tyr-Pro-D-Phe-Thr-OH
Tyr-Pro-D-Phe-Thr-$NH_2$
Tyr-Pro-D-Phe-Thr-Ile-OH
Tyr-Pro-D-Phe-Thr-Thr-OH
Tyr-Pro-Phe-D-Thr-Ile-OH
Tyr-Pro-Phe-D-Thr-Ile-$NH_2$
Tyr-Pro-Phe-D-Thr-Thr-OH
Tyr-Pro-Phe-D-Thr-Thr-$NH_2$
Tyr-Pro-Phe-Gln-OH
Tyr-Pro-Phe-Gln-$NH_2$
Tyr-Pro-Phe-Gly-$NH_2$
Tyr-Pro-Phe-Asp-OH
Tyr-Pro-Phe-Asp-$NH_2$
Tyr-Pro-Phe-Thr-Thr-OH
Tyr-Pro-Phe-Thr-Ser-OH
Tyr-Pro-Phe-Thr-Leu-OH

8. Peptides à activité pharmacologique, suivant l'une des revendications 1–7, caractérisés en ce qu'ils faient des effets en système nerveux central.

9. Peptides à activité pharmacologique, suivant l'une des revendications 1–7, caractérisés en ce qu'ils faient des effets endocrines.

10. Peptides à activité pharmacologique, suivant l'une des revendications 1–7, caractérisés en ce que les peptides faient des effets immunmodulaires.

11. Peptides à activité pharmacologique, suivant l'une des revendications 1–7, caractérisés en ce que les peptides activement le métabolisme cellulaire.

12. Peptides à activité pharmacologique, suivant l'une des revendications 1–7, caractérisés en ce qu'ils peuvent appliqués comme antigènes – après couplage à thyreoglobuline ou à autres corps d'albumine macromoleculaires – à procréation des anticorps dans l'organisme de mammifère.

13. Peptides à activité pharmacologique, suivant l'une des revendications 1–7, caractérisés en ce que les anticorps peuvent appliqués contre des peptides inventives à déterminer les mêmes en tissus de corps et humeurs.

14. Préparations pharmaceutiques, caractérisées en ce qu'elles contiennent des peptides à activité pharmacologique et/ou leurs dérivés et/ou leurs sels d'acide et/ou leurs complexes métallique qu'ils sont définis en revendications 1–11.